(19) Europäisches Patentamt
European Patent Office
Office européen des brevets

(11) Publication number: **0 155 735**
**B1**

(12) # EUROPEAN PATENT SPECIFICATION

(45) Date of publication of patent specification: **19.10.88**

(51) Int. Cl.⁴: **C 07 C 126/02**

(21) Application number: **85200387.0**

(22) Date of filing: **15.03.85**

(54) **Process for the preparation of urea.**

(30) Priority: **16.03.84 NL 8400839**

(43) Date of publication of application:
**25.09.85 Bulletin 85/39**

(45) Publication of the grant of the patent:
**19.10.88 Bulletin 88/42**

(84) Designated Contracting States:
**AT BE DE FR GB IT NL SE**

(56) References cited:
**NL-A-7 007 873**
**US-A-3 356 723**
**US-A-4 013 718**

**EUROPEAN CHEMICAL NEWS UREA SUPPLEMENT, January 17, 1969, pages 17-20; "DSM carbon dioxide stripping process"**

(73) Proprietor: **STAMICARBON B.V.**
**Mijnweg 1**
**NL-6167 AC Geleen (NL)**

(72) Inventor: **Jonckers, Kees**
**Illikhoven 13**
**NL-6121 RH Born (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a process for the preparation of urea from ammonia and carbon dioxide.

If ammonia and carbon dioxide are introduced into a synthesis zone under a suitable pressure (for instance 125—350 atm) and at a suitable temperature (for instance 170—250°C), first ammonium carbamate is formed, according to the reaction:

$$2NH_3 + CO_2 \rightarrow H_2N—CO—ONH_4$$

From the ammonium carbamate thus obtained, subsequently urea is formed by dehydration according to the reversible reaction:

$$H_2N—CO—ONH_4 \rightleftarrows H_2N—CO—NH_2 + H_2O$$

The degree to which the conversion to urea takes place depends among others on the temperature and the ammonia excess used. As reaction product, a solution is obtained that consists mainly of urea, water, ammonium carbamate and free ammonia. The ammonium carbamate and the ammonia are to be removed from the solution and are mostly returned to the synthesis zone. The synthesis zone may consist of separate zones for the formation of carbamate and urea, but these zones may also be accommodated in one apparatus.

A process frequently applied for the preparation of urea is described in European Chemical News, Urea Supplement, of January 17, 1969, pages 17—20. In this process, the urea synthesis solution formed at high pressure and temperature in the synthesis zone is subjected to a stripping treatment at synthesis pressure by countercurrently contacting the solution with gaseous carbon dioxide while supplying heat, so that the larger part of the carbamate present in the solution is decomposed into ammonia and carbon dioxide. These decomposition products are expelled from the solution in gaseous form and discharged together with a small amount of water vapour and the carbon dioxide used for stripping. Such a stripping treatment can be carried out not only with carbon dioxide, as described in this publication, but also with gaseous ammonia, an inert gas or a mixture of at least two of said gases. The gas mixture obtained in the stripping treatment is for the larger part condensed in a condensation zone and absorbed in an aqueous solution originating from the further treatment of the urea-containing solution, following which both the aqueous carbamate solution thus formed and the non-condensed gas mixture are sent to the synthesis zone for urea formation. Here, the heat required for conversion of carbamate to urea is obtained by further condensation of the gas mixture.

In U.S. patent specification 3,356,723 it is stated that there are advantages in having the urea synthesis take place at relatively low pressures of, for instance, 110—140 atmospheres, because this admits to perform the stripping treatment by which the excess ammonia and the carbamate not converted to urea are removed at ther same pressure as the synthesis reaction. If, on the other hand, the synthesis reaction is carried out at substantially higher pressures, for instance about 200 atmospheres, then the pressure of the synthesis solution preferably is reduced before the solution is subjected to the stripping treatment. However, as the pressure at which the urea synthesis solution is stripped becomes lower, more water is required to keep in solution the carbamate formed during condensation at the pressure of the stripping operation of the gas mixture obtained in the stripping treatment, and this water accompanies the carbamate when it enters the synthesis zone, where it adversely affects the synthesis efficiency. In addition, as a consequence of the lower pressure, the condensation of the gas mixture obtained in stripping takes place at a lower temperature, so that the heat of condensation and the heat of dissolution released are to be removed at a relatively low temperature level, necessitating a relatively large heat-exchanging surface. With this low-level heat, only low-pressure steam of 2—3 bar can be produced, for which there is little use, neither in the process, nor elsewhere.

If, on the other hand, stripping is carried out at relatively high pressures and the high temperatures required for this, the risk of urea hydrolysis and biuret formation taking place to an unacceptable degree is not inconceivable. For these reasons, said U.S. patent specification 3,356,723 recommends a pressure of 50—140 atmospheres in the stripping zone.

The object of the present invention is to provide a process for the preparation of urea in which a good synthesis efficiency is achieved and in which urea hydrolysis and biuret formation during the stripping treatment remain within acceptable limits, while furthermore condensation of the gas mixture obtained in the stripping treatment is carried out at such a temperature level that a subtantially smaller heat-exchanging surface can be used for converting the heat liberated into low-pressure steam of, for instance, 3—5 bar, or that steam of higher pressure, for instance 5—10 bar can be produced, or that the heat liberated can be used directly for heating of process flows.

According to the invention this is accomplished in that urea is also allowed to form in the condensation zone during condensation of the gas mixture obtained in the stripping treatment. On account of the presence of relatively large amounts of urea and water, which serve as solvents for the carbamate formed upon condensation of the gas mixture obtained on stripping, the heat of condensation and the heat of dissolution become available at a higher temperature level than without application of these solvents.

The invention therefore relates to a process for the preparation of urea in which a carbamate- and free ammonia-containing urea synthesis solution

is formed from carbon dioxide and excess ammonia in a synthesis zone at a pressure of 125—350 bar, at least a portion of the carbamate present in the urea synthesis solution is decomposed in a stripping zone at the pressure of the synthesis zone or at lower pressure by heat supply and countercurrent contact with a stripping gas, the carbamate decomposition products, together with a portion of the excess ammonia and the stripping gas, are removed from the stripping zone, as a gas mixture, at least a portion of the gas mixture obtained is condensed in a condensation zone and the stripped urea synthesis solution is processed into a urea solution or solid urea. The process is characterized in that in the condensation zone also at least 30% of the equilibrium amount of urea achievable under the reaction conditions is allowed to form and the carbamate- and urea-containing mixture is supplied to the synthesis zone.

When at least 30% of the achievable equilibrium amount of urea has formed, the heat effect can already clearly be noticed. By preference, urea formation is continued until 50—80% of the way to the equilibrium has been covered.

Just as the stripping treatment, condensation of the gas mixture obtained in the stripping treatment can be carried out at the same pressure as the synthesis pressure or at a lower pressure. It is also possible for the pressures at which stripping and condensation are carried out to be equal to or different from each other. By preference equal pressures are applied in the synthesis zone, the stripping zone and the condensation zone, so that the carbamate solution formed in the condensation zone can be returned to the synthesis zone in a non-complicated manner without a carbamate pump.

Conversion of carbamate into urea and water in the condensation zone can be effected by ensuring that the residence time of the reaction mixture in the condensation zone is sufficiently long. Condensation of the gas mixture obtained on stripping, yielding a carbamate solution, and further conversion of carbamate into urea and water may, for instance, be effected on the shell side of a vertical tubular heat exchanger. The heat liberated in the condensation zone can then be carried off by means of water led through the tubes and thereby converted into low-pressure steam or by means of a process flow to be heated. To increase the temperature further, the condensation zone may be designed as a reactor with intensive mixing, the temperature difference between top and bottom being limited to at most 5°C and preferably at most 2°C. Installation of guide plates, baffles or similar elements promotes mixing and thus heat transfer. Use can also be made of other condensation zones having such dimensions as to allow of a sufficiently long residence time of the reaction mixture. By preference, the condensation zone is given the form of a so-called submerged condenser, the gas mixture to be condensed being led into the shell space of a tubular heat exchanger, into which shell space also a dilute carbamate solution is introduced, and the heat of dissolution and the heat of condensation that are liberated are removed with the aid of a medium flowing through tubes, for instance water, which is thereby converted into low-pressure steam. The submerged condenser may be placed horizontally or vertically.

There are special advantages in effecting the condensation in a horizontal submerged condenser. The liquid column as a rule having less height than in a vertical submerged condenser, the elevation of the boiling point is less high, resulting in a larger temperature difference between the urea-containing carbamate solution and the cooling medium, and thus in faster heat transfer.

When a horizontally placed submerged condenser is used, it can be placed directly on the work floor, so that the height of the installation is clearly reduced and the installation costs are decreased. Furthermore, in that case assembly and disassembly are relatively simple.

When a vertical condensation zone is applied, it is possible to house the synthesis zone and the condensation zone in one apparatus, so that a compact construction is obtained.

In the process according to the invention it is possible, depending on the pressure applied in the condensation zone and the amounts of urea and water formed, to raise the temperature in the condensation zone by 5—10°C. This way, it is, for instance, possible to generate low-pressure steam of 5—10 bar at a pressure in the condensation zone of about 140 bar. Of course, it is also possible to produce low-pressure steam having the usual pressure of 3—5 bar. This can then be achieved using a substantially smaller heat-exchanging surface.

The invention will be elucidated with reference to the figure and the examples, without however being restricted thereto.

In the figure, A represents a synthesis zone, B a stripping zone and C a condensation zone. D represents a steam reservoir, E a pump and F an ejector.

Through 7, ejector F, which is driven by means of compressed liquid ammonia supplied through 6, aspirates a carbamate solution obtained in the further processing of the urea-containing solution produced elsewhere in the process. The mixture of liquid ammonia and carbamate solution is fed to condensation zone C through 5. Through line 3, which is provided with openings, a gas mixture containing ammonia and carbon dioxide is introduced into the liquid, said gas mixture having been obtained by subjecting the urea synthesis solution formed in synthesis zone A to a stripping treatment in stripping zone B, which is effected countercurrent to a stripping gas, for instance carbon dioxide, while heat is being supplied. In the mode of realization represented here, the pressure in synthesis zone A, stripping zone B and condensation zone C are equal, for instance approximately 140 bar. However, it is also possible for the pressure in said zones to differ from

one another. The dimensions of condensation zone C have been chosen so that the residence time of the reaction mixture here is sufficiently long, so that at least 30% of the urea formation theoretically possible takes place. The heat liberated in condensation zone C is removed with the aid of water, supplied through 12, which is passed through 10 and through cooling elements 8 in condensation zone C by means of pump E, and which is thereby converted into low-pressure steam. The steam formed is passed through 9 into steam reservoir D and through 11 discharged from said reservoir to a plant consuming low-pressure steam, which is not depicted. Instead of removing the heat by means of steam generation, a process flow to be heated, for instance an aqueous urea solution that is to be concentrated, can be led through the cooling elements. The urea- and carbamate-containing solution and the non-condensed portion of the gas mixture fed to condensation zone C are supplied through 4 to synthesis zone A, where further urea formation takes place.

From synthesis zone A, the urea synthesis solution is passed through 1 to stripping zone B, while a gas mixture containing the inert gases is removed through 14. The stripped urea synthesis solution is discharged through 13, in a known way processed into an aqueous urea solution and concentrated, following which the concentrated solution may be converted into solid urea.

Comparative Example

Urea is prepared by the process described in European Chemical News, Urea Supplement of January 17, 1969, in an amount of 1500 tons a day. The amounts are given in kg per hour. The high-pressure section of the plant is supplied with 33,417 kg liquid $NH_3$ and 45,833 kg gaseous $CO_2$, which also contains 286 kg water vapour and 1,533 kg inert components. The pressure in the reaction zone, the stripping zone and the condensation zone is 141.2 bar. To the condensation zone a gas mixture is supplied that consists of 43,742 kg $NH_3$, 65,795 kg $CO_2$, 2,578 kg $H_2O$ and 1,533 kg inert components, which is for the larger part condensed here and absorbed in a carbamate solution containing 50,964 kg $NH_3$, 17,957 kg $CO_2$ and 9,231 kg $H_2O$. A carbamate solution is formed that consists of 78,108 kg $NH_3$, 67,706 kg $CO_2$ and 10,829 kg $H_2O$ and has a temperature of 168°C, while the remaining gas mixture contains 18,598 kg $NH_3$, 16,046 kg $CO_2$, 980 kg $H_2O$ and 1,533 kg inerts. The carbamate solution and the gas mixture are supplied to the reaction zone.

The heat liberated on condensation is removed with the aid of water, resulting in the formation of 60,000 kg low-pressure steam of 4.4 bar and 147°C. The heat-exchanging surface is 1,694 $m^2$.

Example I

In a plant as depicted in the figure the process according to the invention is used to prepare the same amount of urea as described in the comparative example. The pressure in the high-pressure section of the plant is 141.2 bar. The condensation of the gas mixture obtained on stripping is effected in a horizontally placed submerged condenser, which is accompanied by the formation of 50% of the amount of urea theoretically possible under the prevailing conditions. The urea-containing carbamate solution formed consists of 30,839 kg urea, 60,871 kg $NH_3$, 50,553 kg $CO_2$ and 20,521 kg $H_2O$. Its temperature is 174°C. In addition, a gas mixture remains that consists of 18,171 kg $NH_3$, 10,706 kg $CO_2$, 606 kg $H_2O$ and 1,533 kg inert components. With the aid of the heat liberated in the condensation zone, 60,000 kg low-pressure steam of 4.4 bar is formed. The heat-exchanging surface required for this is 667 $m^2$.

Example II

In the way described in Example I, an identical amount of urea is formed, but now in the condensation zone 75% of the achievable equilibrium amount of urea is formed. In the condensation zone a solution is obtained that contains, besides 56,188 kg $NH_3$ and 40,203 kg $CO_2$, also 47,930 kg urea and 25,649 kg $H_2O$.

In addition, a gas mixture remains that consists of 14,825 kg $NH_3$, 8,400 kg $CO_2$, 605 kg $H_2O$ and 1,533 kg inert components. The temperature of the liquid and gaseous components discharged from the condensation zone is 178°C. The liberated heat is utilized for the formation of 60,000 kg low-pressure steam of 6.8 bar.

The heat-exchanging surface required for this is 1,694 $m^2$.

**Claims**

1. Process for the preparation of urea in which a carbamate and free ammonia containing urea synthesis solution is formed from carbon dioxide and excess ammonia in a synthesis zone at a pressure of 125—350 bar, at least a portion of the carbamate present in the urea synthesis solution is decomposed in a stripping zone at the pressure of the synthesis zone or at a lower pressure by heat supply and counter-current contact with a stripping gas, the carbamate decomposition products, together with a portion of the excess ammonia and the stripping gas, are removed from the stripping zone as a gas mixture, at least a portion of the gas mixture obtained is condensed in a condensation zone and the stripped urea synthesis solution is processed into a urea solution or solid urea, this process being characterized in that the residence time of the reaction mixture in the condensation zone is sufficiently long that also at least 30% of the equilibrium amount of urea achievable under the reaction conditions is allowed to form and the carbamate- and urea-containing mixture is supplied to the synthesis zone.

2. Process according to claim 1, characterized in that 50—80% of the equilibrium amount of urea achievable under the prevailing conditions is allowed to form.

3. Process according to claim 1 or 2, characterized in that the condensation in the condensation zone is carried out at synthesis pressure.

4. Process according to claim 1 or 2, characterized in that the condensation is effected at the pressure at which the stripping treatment takes place, which pressure is lower than the synthesis pressure.

5. Process according to any of claims 1—4, characterized in that it is ensured that the reaction mixture is thoroughly mixed in the condensation zone.

6. Process according to any of claims 1—5, characterized in that the condensation is effected in a submerged condenser.

7. Process according to any of claims 1—6, characterized in that the condensation is effected on the shell side of a horizontally placed tubular heat exchanger.

8. Process according to any of claims 1—6, characterized in that the condensation is effected in a condensation zone integrated with the reaction zone.

**Patentansprüche**

1. Verfahren zur Herstellung von Harnstoff, wobei eine Carbamat und freien Ammoniak enthaltende Harnstoffsyntheselösung aus Kohlendioxid und überschüssigem Ammoniak in einer Synthesezone bei einem Druck von 125 bis 350 bar gebildet wird, wenigstens ein Teil des Carbamats, das in der Harnstoffsyntheselösung vorhanden ist, in einer Abstreifzone bei dem Druck der Synthesezone oder bei niedrigerem Druck durch Wärmezufuhr und Gegenstromkontakt mit mit einem Abstreifgas zersetzt wird, die Carbamat-Zersetzungsprodukte zusammen mit einem Teil des überschüssigen Ammoniaks und dem Abstreifgas aus der Abstreifzone als Gasgemisch entfernt werden, wenigstens ein Teil des erhaltenen Gasgemisches in einer Kondensationszone kondensiert werden und die abgestreifte Harnstoffsyntheselösung in eine Harnstofflösung oder festen Harnstoff verarbeitet wird, dadurch gekennzeichnet, dass die Verweilzeit des Reaktionsgemisches in der Kondensationszone genügend lang ist dass auch die Bildung von wenigstens 30% der Gleichgewichtsmenge an Harnstoff, die unter den Reaktionsbedingungen erreichbar ist, ermöglicht wird und die carbamat- und harnstoffhaltige Mischung der Synthesezone zugeführt wird.

2. Verfahren gemäß Anspruch 1, dadurch gekennzeichnet, daß die Bildung von 50—80% der Gleichgewichtsmenge an Harnstoff, die unter den herrschenden Reaktionsbedingungen erreichbar ist, ermöglicht wird.

3. Verfahren gemäß Anspruch 1 oder 2, dadurch gekennzeichnet, daß die Kondensation in der Kondensationszone bei Synthesedruck durchgeführt wird.

4. Verfahren gemäß Anspruch 1 oder 2, dadurch gekennzeichnet, daß die Kondensation bei dem Druck durchgeführt wird, bei dem die Abstreifbehandlung stattfindet, wobei dieser Druck niedriger ist als der Synthesedruck.

5. Verfahren gemäß einem der Ansprüche 1 bis 4, dadurch gekennzeichnet, daß sichergestellt wird, daß das Reaktionsgemisch in der Kondensationszone gründlich vermischt wird.

6. Verfahren gemäß einem der Ansprüche 1 bis 5, dadurch gekennzeichnet, daß die Kondensation in einem Eintauchkondensator durchgeführt wird.

7. Verfahren gemäß einem der Ansprüche 1 bis 6, dadurch gekennzeichnet, daß die Kondensation auf der Mantelseite eines horizontal angeordneten Röhren-Wärmeaustauschers durchgeführt wird.

8. Verfahren gemäß einem der Ansprüche 1 bis 6, dadurch gekennzeichnet, daß die Kondensation in einer mit der Reaktionszone integrierten Kondensationszone durchgeführt wird.

**Revendications**

1. Procédé de préparation d'urée dans lequel on forme une solution de synthèse d'urée contenant du carbamate et de l'ammoniac libre dans une zone de synthèse (A) sous une pression de 125 à 350 bars; on décompose au moins une portion du carbamate présent dans la solution de synthèse d'urée dans une zone de strippage (B) à la pression de la zone de synthèse (A) ou une pression plus basse par apport de chaleur et contact à contre-courant avec un gaz de strippage; on enlève de la zone de strippage (B) les produits de décomposition du carbamate ensemble avec une portion de l'excès d'ammoniac et le gaz de strippage, sous forme d'un mélange gazeux; on condense au moins une partie du mélange gazeux obtenu dans une zone de condensation (C); et on traite la solution strippée de synthèse d'urée pour former une solution d'urée ou une urée solide, procédé caractérisé en ce que la durée de séjour du mélange de réaction dans la zone de condensation (C) est suffisant longue pour permettre de se former également au moins 30% de la quantité d'équilibre d'urée qui peut être obtenue dans les conditions de réaction et on fournit le mélange contenant le carbamate et l'urée à la zone de synthèse (A).

2. Procédé selon la revendication 1, caractérisé en ce qu'on laisse se former de 50 à 80% de la quantité d'équilibre d'urée possible dans les conditions en vigueur.

3. Procédé selon la revendication 1 ou 2, caractérisé en ce que la condensation dans la zone de condensation (C) est effectuée à la pression de synthèse.

4. Procédé selon la revendication 1 ou 2, caractérisé en ce qu'on effectue la condensation sous une pression à laquelle a lieu le traitement de strippage, pression qui est plus basse que la pression de synthèse.

5. Procédé selon l'une quelconque des revendications 1 à 4, caractérisé en ce qu'on assure que le mélange de réaction soit intimement mélangé dans la zone de condensation (C).

6. Procédé selon l'une quelconque des revendications 1 à 5, caractérisé en ce qu'on effectue la condensation dans un condenseur submergé.

7. Procédé selon l'une quelconque des revendications 1 à 6, caractérisé en ce qu'on effectue la condensation sur le côté enveloppe d'un échangeur de chaleur tubulaire disposé horizontalement.

8. Procédé selon l'une quelconque des revendications 1 à 6, caractérisé en ce qu'on effectue la condensation dans une zone de condensation (C) intégrée à la zone de réaction (A).

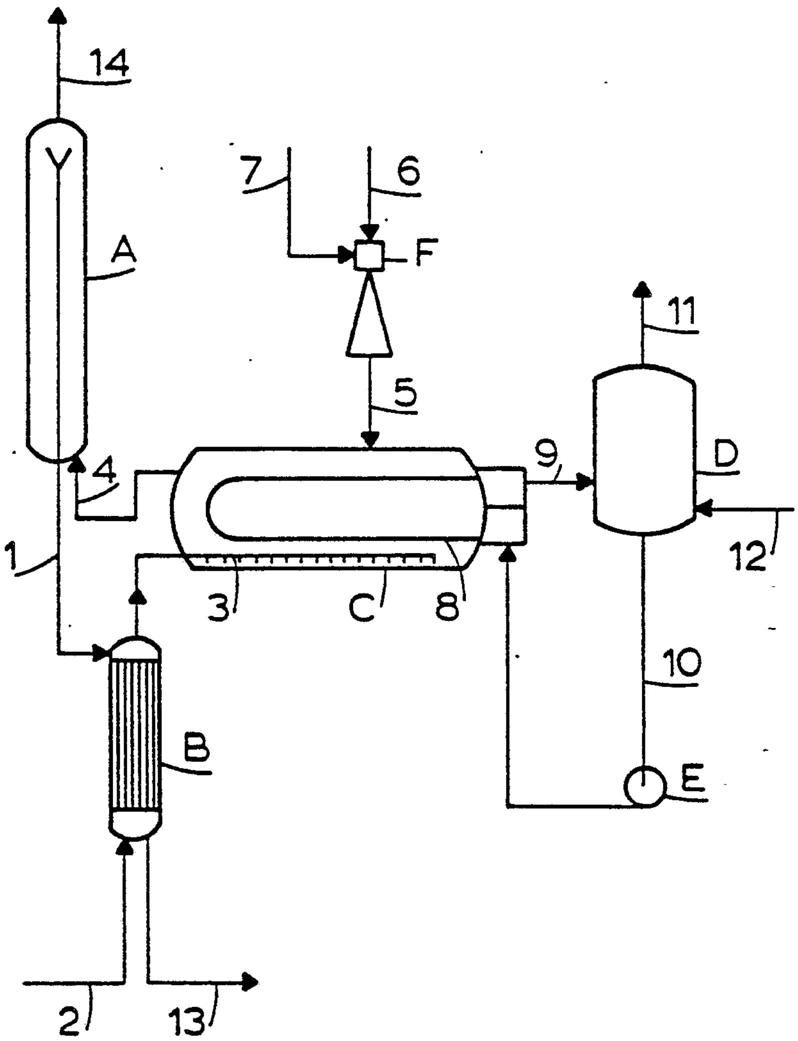
14
A
7
6
F
11
5
9
D
4
1
3
C
8
12
10
B
E
2
13